# EUROPEAN PATENT SPECIFICATION

(11) **EP 2 410 316 B1**
(45) Date of publication and mention of the grant of the patent: **04.03.2015**
(21) Application number: 11173116.2
(22) Date of filing: 07.07.2011
(51) Int. Cl.: G01N 21/89, D01H 13/26, G01N 33/36, B65H 63/06

(54) **Textile material measuring device and yarn winding machine**
Textilmaterialmessgerät und Garnwickelmaschine
Dispositif de mesure de matériaux textiles et dispositif de bobinage de fil

(30) Priority: 23.07.2010 JP 2010166033
(43) Date of publication of application: 25.01.2012
(73) Proprietor: Murata Machinery, Ltd., Minami-ku Kyoto-shi Kyoto 601-8326 (JP)
(72) Inventor: Nakade, Kazuhiko, Kyoto, 612-8686 (JP)
(74) Representative: Stöckeler, Ferdinand

(56) References cited:
- EP-A1- 1 058 112
- EP-A1- 1 508 798
- EP-A1- 1 808 690
- EP-A2- 0 399 945
- US-A- 5 915 279
- US-B1- 6 201 602

## Description

### BACKGROUND OF THE INVENTION

### 1. Field of the Invention

The present invention mainly relates to a textile material measuring device for detecting a foreign particle included in a textile material.

### 2. Description of the Related Art

There are cases where a device adapted to wind a textile material such as a spun yarn includes a textile material measuring device for detecting a foreign particle included in the textile material or thickness unevenness of the textile material. As a configuration for detecting thickness unevenness of a spun yarn and a foreign particle mixed in the spun yarn, for example, there has been generally known an optical measuring device adapted to detect the thickness unevenness in accordance with a change of transmitted light caused by a near-infrared Light Emitting Diode (LED) and detect the foreign particle in accordance with a change of reflection of light irradiated to the spun yarn by the use of a visible light LED. The amount of both the transmitted light and the reflected light is electrically converted by a light receiving element such as a photo diode.

The detection of a foreign particle by the reflected light is carried out by using a feature that optical reflectivity is different between a textile material and a foreign particle. For example, if a colored foreign particle is included in a white spun yarn, the amount of reflected light is reduced at a portion where the colored foreign particle is present; therefore, the foreign particle can be detected by measuring a change of such reflected light.

Sensitivity for detecting a foreign particle by the visible light LED depends on the color of the foreign particle and the color of a light source. For example, when irradiating green light to a spun yarn by a green LED, the green light indicates high sensitivity for detecting a red foreign particle being complementary color of the green light, but indicates low sensitivity for detecting a green foreign particle. Likewise, when irradiating yellow light to the spun yarn by a yellow LED, the yellow light indicates high sensitivity for detecting a blue foreign particle being complementary color with respect to the yellow light, but indicates low sensitivity for detecting a yellow foreign particle. Conventionally, since there have been needs for increasing the sensitivity for detecting the red foreign particle which is easily noticeable, and the green LED has been easily-available and easily obtained, the detection of a foreign particle has been carried out mainly by the green LED. Accordingly, a conventional textile material measuring device indicates low sensitivity for detecting a green foreign particle.

Meanwhile, Japanese Unexamined Patent Application Publication No. 2007-212423 discloses a foreign particle detecting device which is structured to prevent removing a trash by using in an opposite manner, a feature that sensitivity for detecting a yellow foreign particle is reduced by the use of a yellow LED. That is, the trash (i.e., cotton stems and leaves) is a foreign particle which is not required to be removed. By detecting a foreign particle by yellow light similar in color to the trash, the trash can be prevented from being easily detected as the foreign particle.

As described above, in the detection of a foreign particle by the conventional textile material measuring device, there has been a limit to the color of a detectable foreign particle. Japanese Unexamined Patent Application Publication No. 2007-212423 takes advantage of such a feature; however, when a foreign particle required to be removed is yellow, a problem that the detection of such a foreign particle is difficult has not been solved yet.

EP 0 399 945 2 describes a method and an apparatus for the detection of contaminations, impurities and fibers in textile fiber materials. The fiber material is illuminated with polychromatic light. At least two frequencies of the reflected light are selectively detected and compared. The output signal of a difference circuit used for this purpose is kept constant by a closed loop control circuit. As a consequence of a large acting time constant, foreign fibers produce a spontaneous change in the output signal if they differ in color from the raw cotton.

EP 1 508 798 A1 describes an approach for detecting foreign material mixed into a yarn. A yarn is irradiated with ultraviolet rays alternately emitted by light sources. Light receiving elements then alternately receive a light reflected by the yarn. Sample-and-hold circuits hold signals corresponding to the quantities of light received. An adder adds the signals for the quantities of light reflected together. The mixture of foreign material is detected on the basis of a change in the added signal.

### SUMMARY OF THE INVENTION

It is an object of the present invention to provide a textile material measuring device capable of detecting foreign particles of all the colors and thickness unevenness in a textile material.

This object is achieved by a textile material measuring device according to claim 1.

According to a first aspect of the present invention, the textile material measuring device includes a first light source section, a second light source section, a first light receiving section, a second light receiving section, a first detecting section, and a second detecting section. The first light source section is adapted to irradiate first-color light to a textile material. The second light source section is adapted to irradiate second-color light to the textile material, the second-color light being complementary color with respect to the first-color light. The first light receiving section is adapted to receive reflected light, the reflected light being the light from the first light source section reflected by the textile material. The second light receiving section is adapted to receive reflected light and transmitted light, the reflected light being the light from the second light source section reflected by the textile material, and the transmitted light being the light from the second light source section that has been transmitted through the textile material. The first detecting section is adapted to detect a foreign particle in the textile material in accordance with the reflected light received by the first light receiving section and the second light receiving section. The second detecting section is adapted to detect thickness of the textile material in accordance with the transmitted light received by the second light receiving section.

As described above, by the use of light of two different colors of which one is complementary color with respect to the other, even if sensitivity for detecting a foreign particle of a particular color is reduced by light from one of two light sources, such a foreign particle can be detected by light from the other light source. Since weakness with respect to particular colors can be complemented by the two light sources as described above, the above-described textile material measuring device can detect foreign particles of all colors at excellent sensitivity. Further, by using one of the two light sources as a light source for detecting yarn thickness, the textile material detecting device can be structured to be simple so that costs can be reduced.

It is preferable in the above-described textile material measuring device that the first-color light irradiated by the first light source section is yellow, and the second-color light irradiated by the second light source section is blue. Since polypropylene has a property indicating high reflectivity with respect to blue light, the textile material measuring device can detect polypropylene using the blue light source as described above. By detecting a foreign particle by the use of a yellow light source being complementary color of blue, the textile material measuring device can successfully detect a blue foreign particle which is difficult to be detected by the blue light source.

According to a second aspect of the present invention, the textile material measuring device includes the first light source section, the second light source section, the first light receiving section, the second light receiving section, the first detecting section, and the second detecting section. The first light source section is adapted to irradiate yellow light to a textile material. The second light source section is adapted to irradiate ultraviolet light to the textile material. The first light receiving section is adapted to receive reflected light, the reflected light being the light from the first light source section reflected by the textile material. The second light receiving section is adapted to receive reflected light and transmitted light, the reflected light being the light from the second light source section reflected by the textile material, and the transmitted light being the light from the second light source section that has been transmitted through the textile material. The first detecting section is adapted to detect a foreign particle in the textile material in accordance with the reflected light received by the first light receiving section and the second light receiving section. The second detecting section is adapted to detect thickness of the textile material in accordance with the transmitted light received by the second light receiving section.

Since polypropylene has a property indicating high reflectivity with respect to ultraviolet light, the textile material measuring device can detect polypropylene using a light source of ultraviolet light as described above. Meanwhile, it is difficult to detect a blue foreign particle by the above-described light source of ultraviolet light having the similar wavelength as blue light. Accordingly, by detecting a foreign particle by the use of a yellow light source being complementary color of blue, the textile material measuring device can successfully detect a blue foreign particle which is difficult to be detected by the ultraviolet light.

In the above-described textile material measuring device, the first detecting section preferably includes a distinguishing section adapted to compare a measured value of the reflected light with a threshold value to distinguish a trash and a foreign particle, other than the trash, mixed in the textile material. Since the trash has a color which is difficult to be detected by a yellow light source, by comparing reflected light from the yellow light source with reflected light from a light source other than the yellow light source, the textile material measuring device can distinguish whether or not the detected foreign particle is a trash.

When using a light source of visible color as the first light source section and the second light source section, the above-described textile material measuring device preferably further includes a resinous housing adapted to accommodate the first light source section, the second light source section, the first light receiving section, and the second light receiving section. Since visible light does not deteriorate resin, the light source sections can be accommodated in the resinous housing. By accommodating the two light source sections and the two light receiving sections in a single housing, the textile material measuring device can be formed compact.

In the above-described textile material measuring device, the first light receiving section is adapted to receive transmitted light in addition to the reflected light, the transmitted light being the light from the first light source section that has been transmitted through the textile material. In a travelling direction of the textile material, the first light source section and the first light receiving section are arranged at prescribed interval at upstream of the second light source section and the second light receiving section. The textile material measuring device includes a travelling speed acquiring section adapted to acquire travelling speed of the textile material in accordance with the transmitted light received by the first light receiving section and the second light receiving section and the prescribed interval.

When measuring light transmitted through the travelling textile material at upstream of the travelling direction of the textile material and at downstream thereof, there is a time delay in a measured value at downstream thereof when comparing with a measured value at upstream thereof. Therefore, the textile material measuring device can calculate the travelling speed of the textile material by detecting such a time delay.

In the textile material measuring device, the first light source section includes two light emitting elements that alternately emits light. The first light receiving section includes two light receiving elements that receive reflected light and transmitted light. The second light source section includes a single light emitting element. The second light receiving section includes a single light receiving element that receives transmitted light.

That is, the first light source section and the first light receiving section are structured as a double-axis measuring section, and the second light source section and the second light receiving section are structured as a single-axis measuring section. The double-axis measuring section can irradiate light to a textile material from a plurality of directions; therefore, excellent detection sensitivity can be maintained even if a cross-sectional shape of the textile material is flat. Further, since a transmitted light receiving element perpendicular to a light axis is arranged in the single-axis measuring section, detection range of transmitted light is wide. Each of the single-axis measuring section and the double-axis measuring section has a feature described above; therefore, by combining such two types of measuring sections, detection performance of the textile material measuring device can be improved.

According to a third aspect of the present invention, a yarn winding machine includes a winding device adapted to wind a yarn into a package and the textile material measuring device adapted to measure the yarn that is travelling. Accordingly, the yarn winding machine can form a high-quality package in which a foreign particle is removed and an abnormality in yarn thickness is solved.

### BRIEF DESCRIPTION OF THE DRAWINGS

FIG. 1 is a side view of a winder unit of an automatic winder according to an embodiment of the present invention.
FIG. 2 is a schematic front view of the winder unit.
FIG. 3 is a plane cross-sectional view of a clearer head illustrating a configuration of a first measuring section.
FIG. 4 is a plane cross-sectional view of the clearer head illustrating a configuration of a second measuring section.

### DETAILED DESCRIPTION OF PREFERRED EMBODIMENT

A preferred embodiment of the present invention will be described below with reference to the drawings.

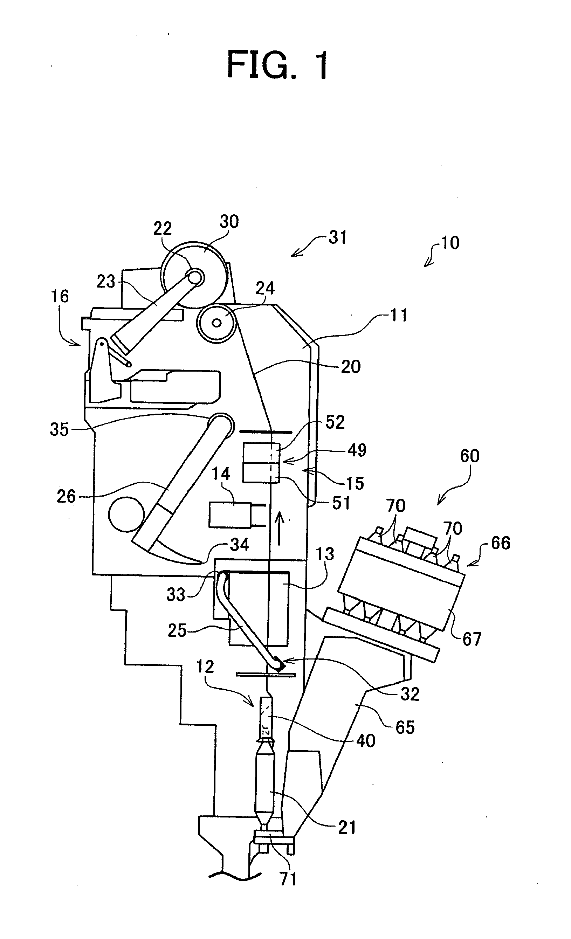

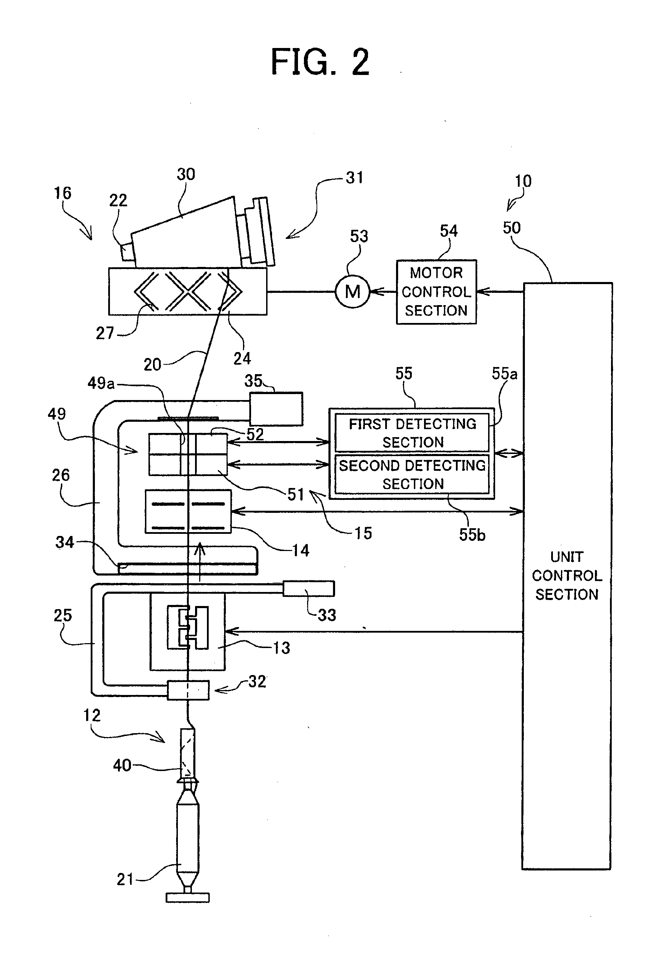

A winder unit 10 illustrated in FIG. 1 and FIG. 2 winds a spun yarn (textile material) 20 unwound from a yarn supplying bobbin 21 around a winding bobbin 22 while traversing the spun yarn 20, in order to form a package 30 with a prescribed shape and a prescribed length. An automatic winder (yarn winding machine) according to the present embodiment includes a plurality of winder units 10 which are arranged in line and a machine control device (not illustrated in the drawings) which is arranged in one end in a direction in which the winder units 10 are arranged.

Each of the winder units 10 includes a unit frame 11 (illustrated in FIG. 1) arranged at either a right side thereof or a left side thereof when viewed from a front side, and a winding unit main body 16 arranged at a lateral side of the unit frame 11. The winding unit main body 16 includes a magazine-typed supply device 60, a winding section 31, and a yarn supplying bobbin holding section 71.

As illustrated in FIG. 1, the magazine-typed supply device 60 includes a magazine holding section 65 and a bobbin storing device 66. The magazine holding section 65 extends diagonally upward from a lower part of the winder unit 10 at a front side of the winder unit 10. The bobbin storing device 66 is attached to a tip end of the magazine holding section 65. The bobbin storing device 66 includes a magazine can 67 in which a plurality of storage holes are circularly formed. A supply bobbin 70 can be set in each of the storage holes in an inclined posture. The magazine can 67 can be intermittently driven, rotated, and fed by a motor (not illustrated in the drawings). By such intermittent driving and a control valve (not illustrated in the drawings) of the magazine can 67, the supply bobbin 70 can be dropped one by one to a bobbin feeding pathway (not illustrated in the drawings) of the magazine holding section 65. The supply bobbin 70 supplied to the bobbin feeding pathway is fed to the yarn supplying bobbin holding section 71, remaining in an inclined posture.

The yarn supplying bobbin holding section 71, which includes a swinging mechanism (not illustrated in the drawings), receives the supply bobbin 70 from the bobbin feeding pathway, and then swings the supply bobbin 70 such that the supply bobbin 70 remaining in an inclined posture stands up substantially upright. Accordingly, the yarn supplying bobbin holding section 71 can hold the fed yarn supplying bobbin 21 substantially upright. Further, in place of the magazine-typed supply device 60 as illustrated in FIG. 1, the yarn supplying bobbin 21 may be fed from a yarn supplying bobbin supply section (not illustrated in the drawings) to the yarn supplying bobbin holding section 71 of each of the winder units 10 by a conveyor (not illustrated in the drawings) arranged in a lower part of the automatic winder.

The winding section 31 winds the spun yarn 20 unwound from the yarn supplying bobbin 21 around the winding bobbin 22 in order to form the package 30. Specifically, the winding section 31 includes a cradle 23 and a winding drum 24. The cradle 23 can hold the winding bobbin 22. The winding drum 24 traverses the spun yarn 20 and drives the winding bobbin 22. The cradle 23 is structured capable of swinging in a direction in which the cradle 23 moves close to or away from the winding drum 24. Accordingly, the package 30 is brought into contact with the winding drum 24 or is separated from the winding drum 24. As illustrated in FIG. 2, a spiral-shaped traverse groove 27 is formed on an outer peripheral surface of the winding drum 24. The spun yarn 20 is traversed by the traverse groove 27.

The winding drum 24 is arranged so as to face the winding bobbin 22. The winding bobbin 22 is driven and rotated by driving and rotation of the winding drum 24. While being traversed by the traverse groove 27, the spun yarn 20 is wound around the rotating winding bobbin 22. As illustrated in FIG. 2, the winding drum 24 is connected with an output shaft of a drum driving motor 53, and the operation of the drum driving motor 53 is controlled by a motor control section 54. The motor control section 54 receives a control signal from a unit control section (controller) 50 to control the operation of the drum driving motor 53 to start or stop.

In the winding unit main body 16, an unwinding assisting device 12, a tension applying device 13, a yarn splicing device 14, and a clearer head 49 of a clearer (textile material measuring device) 15 are arranged in this order from the yarn supplying bobbin 21 side in a yarn travelling pathway between the yarn supplying bobbin 21 and the winding drum 24.

By lowering a regulating member 40 covering a core tube, accompanying unwinding of the spun yarn 20 from the yarn supplying bobbin 21, the unwinding assisting device 12 assists the unwinding of the spun yarn 20 from the yarn supplying bobbin 21. The regulating member 40 makes contact with a balloon formed at an upper part of the yarn supplying bobbin 21 by the spun yarn 20 unwound from the yarn supplying bobbin 21 being swung, and then applies appropriate tension to such a balloon to assist the unwinding of the spun yarn 20.

The tension applying device 13 applies prescribed tension to the travelling spun yarn 20. As the tension applying device 13, a gate-typed device in which movable comb teeth are arranged with respect to fixed comb teeth can be used, for example. The movable comb teeth can be swung by a rotary solenoid such that both comb teeth are engaged with or disengaged from one another. The tension applying device 13 applies constant tension to the spun yarn 20 to be wound into the package 30, thereby improving the quality of the package 30. Further, in addition to the above-described gate-typed device, a disc-typed device can be selected as the tension applying device 13, for example.

The clearer 15 includes the clearer head 49 and an analyzer 55 (illustrated in FIG. 2). The spun yarn 20 travels while passing through the clearer head 49. Measuring sections 51 and 52, which will be described later, are arranged in the clearer head 49 and structured to measure a state of the spun yarn 20. Measurement results of the measuring sections 51 and 52 are transmitted to the analyzer 55. The analyzer 55, which is structured as a computer including a Central processing Unit (CPU) , a Read Only Memory (ROM), a Random Access Memory (RAM), and the like, detects thickness unevenness of the spun yarn 20 or a foreign particle therein in accordance with the measurement results transmitted from the measuring sections 51 and 52. When detecting a portion in the spun yarn 20 including an abnormality in thickness or a foreign particle required to be removed, the analyzer 55 recognizes such a portion as a yarn defect, and then transmits a yarn defect detection signal to the unit control section 50.

A cutter (not illustrated in the drawings) is arranged in the vicinity of the clearer head 49. When the analyzer 55 detects a yarn defect, the unit control section 50 immediately controls the cutter to cut the spun yarn 20. The portion including the yarn defect is removed by such a cutting operation and a yarn splicing operation which will be described later.

When the spun yarn 20 is cut after the clearer 15 detects a yarn defect, or when a yarn breakage occurs during unwinding of the spun yarn 20 from the yarn supplying bobbin 21, or the like, the yarn splicing device 14 splices a lower yarn from the yarn supplying bobbin 21 and an upper yarn from the package 30. As the yarn splicing device 14, a mechanical device, a device using fluid such as compressed air, or the like can be used.

A lower yarn guiding pipe 25 is provided at a lower side of the yarn splicing device 14 to catch and guide the lower yarn from the yarn supplying bobbin 21. An upper yarn guiding pipe 26 is provided at an upper side of the yarn splicing device 14 to catch and guide the upper yarn from the package 30. A suction opening 32 is formed at a tip end of the lower yarn guiding pipe 25. A suction mouth 34 is provided at a tip end of the upper yarn guiding pipe 26. Appropriate negative-pressure sources are connected to the lower yarn guiding pipe 25 and the upper yarn guiding pipe 26, respectively. Thus, suction airflow can act on the suction opening 32 and the suction mouth 34.

In such a configuration, the suction opening 32 of the lower yarn guiding pipe 25 catches the lower yarn at a position illustrated in FIG. 1 and FIG. 2 at the time of a yarn breakage or a yarn cut, and then swings upward around a shaft 33 to guide the lower yarn to the yarn splicing device 14. At approximately the same time, the upper yarn guiding pipe 26 swings upward around a shaft 35 from a position illustrated in the drawings, and then catches by the suction mouth 34 the upper yarn unwound from the package 30 rotated backward by the drum driving motor 53. Subsequently, by swinging downward around the shaft 35, the upper yarn guiding pipe 26 guides the upper yarn to the yarn splicing device 14. Then, the lower yarn and the upper yarn are spliced together by the yarn splicing device 14.

Next, a configuration of the clearer 15 will be described in detail.

In the clearer 15 according to the present embodiment, the optical measuring sections 51 and 52 for detecting a foreign particle included in the spun yarn 20 and thickness unevenness of the spun yarn 20 are arranged one above the other along a travelling direction of the spun yarn 20. Light being used for measurement in the first measuring section 51 is complementary color with respect to light being used for measurement in the second measuring section 52.

That is, as described above, sensitivity for detecting a foreign particle by the clearer 15 depends on the color of light which is irradiated to the spun yarn 20 by the clearer 15. That is, when the irradiated light is complementary color with respect to a foreign particle, the irradiated light indicates high sensitivity for detecting the foreign particle; when the irradiated light is similar color with respect to the color of the foreign particle, the irradiated light indicates low sensitivity for detecting the foreign particle. Accordingly, a conventional clearer, which detects a foreign particle by the use of a light source irradiating single-color light, has limitations on a detectable color range of foreign particles.

Meanwhile, in the clearer 15 according to the present embodiment, the two measuring sections 51 and 52 for detecting a foreign particle are arranged one above the other as described above, and each of the measuring sections 51 and 52 detects a foreign particle by light of different colors. Further, light being used in one of the two measuring sections 51 and 52 is set so as to be complementary color with respect to light being used in the other thereof. Accordingly, a foreign particle incapable of being detected by the light of one of the two colors can be detected by the other thereof. As a result, the clearer 15 can detect foreign particle of all colors; therefore, sensitivity for detecting a foreign particle by the clearer 15 is dramatically improved.

As long as light being used to detect a foreign particle in the measuring section 51 is complementary color with respect to light being used to detect the foreign particle in the measuring section 52, any color combination may be used. In the clearer 15 according to the present embodiment, a color combination of blue and yellow is selected since this color combination is capable of improving sensitivity for detecting polypropylene and recognizing presence of a trash. Such a feature will be described later.

Further, sensitivity for detecting thickness of the spun yarn 20 by the clearer 15 is less influenced by the color of light which is used for such detection. The clearer 15 according to the present embodiment detects a foreign particle and yarn thickness at the same time; therefore, detection of the thickness of the spun yarn 20 will be also described below.

First, a clearer head 49 will be described. The clearer head 49 is structured as a resinous housing. Inside the clearer head 49, each of sections (i.e., a first light source section 61, a first light receiving section 62, a second light source section 63, a second light receiving section 64, and the like which are described later) of the measuring sections 51 and 52 is arranged. In the clearer head 49, a yarn travelling pathway 49a adapted to pass the spun yarn 20 through is formed along a travelling direction of the spun yarn 20. Further, the yarn travelling pathway 49a is formed so as to be opened at a front side of the clearer head 49.

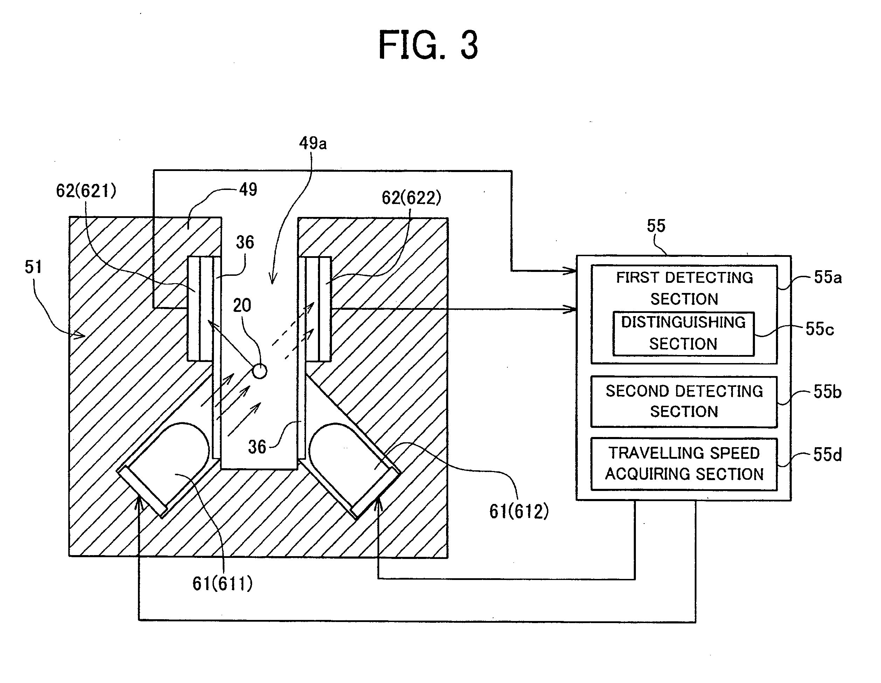

Next, the first measuring section 51 will be described. As illustrated in FIG. 2, the first measuring section 51 is arranged upstream of the second measuring section 52 in the yarn travelling direction. FIG. 3 is a schematic cross-sectional view illustrating a configuration of the first measuring section 51 inside the clearer head 49 of when the clearer head 49 is cut in a plane perpendicular to the yarn travelling direction.

The first measuring section 51 includes the first light source section 61 and the first light receiving section 62. As illustrated in FIG. 3, the first light source section 61 includes two light emitting elements 611 and 612, and the first light receiving section 62 includes two light receiving elements 621 and 622. Since the two light emitting elements 611 and 612 are structured as yellow LEDs, yellow light is irradiated to the spun yarn 20 travelling along the yarn travelling pathway 49a. Meanwhile, the light receiving elements 621 and 622, which are structured as photodiodes, converts intensity of received light into an electric signal to transmit the electric signal to the analyzer 55. This type of measuring section is referred to as a double-axis measuring section since the measuring section is provided with two light emitting elements.

Further, the yarn travelling pathway 49a is separated from the first light source section 61 and the first light receiving section 62 by a resinous transparent plate 36. The first light source section 61 irradiates light to the spun yarn 20 through the transparent plate 36, and the first light receiving section 62 receives the light through the transparent plate 36. By separating the yarn travelling pathway 49a from the first light source section 61 and the first light receiving section 62, yarn waste and the like generated by travelling of the spun yarn 20 along the yarn travelling pathway 49a can be prevented from adhering to the first light source section 61 and the first light receiving section 62; therefore, maintenance of the clearer head 49 is improved.

Each of the first light emitting element 611 and the second light emitting element 612 is arranged so as to irradiate light at a different angle to the spun yarn 20 travelling along the yarn travelling pathway 49a. The analyzer 55 controls the first light emitting element 611 and the second light emitting element 612 to alternatively emit light. Accordingly, light can be irradiated to the spun yarn 20 from two different directions. That is, such a double-axis measuring section can measure the spun yarn 20 from various angles. Therefore, even if a cross-sectional shape of the spun yarn 20 is flat in a cross-sectional view of FIG. 3, for example, accuracy of detecting a foreign particle and yarn thickness can be improved.

The first light receiving element 621 is arranged opposite the second light emitting element 612 across a travelling path of the spun yarn 20. Accordingly, the first light receiving element 621 receives transmitted light, the transmitted light being the light irradiated from the second light emitting element 612 to the spun yarn 20 that has been transmitted through the spun yarn 20. Meanwhile, the second light receiving element 622 is arranged opposite the first light emitting element 611 across the travelling path of the spun yarn 20. Accordingly, the second light receiving element 622 receives transmitted light, the transmitted light being the light irradiated from the first light emitting element 611 to the spun yarn 20 that has been transmitted through the spun yarn 20.

The first light receiving element 621 is arranged so as not to directly receive light from the first light emitting element 611 and arranged capable of receiving reflected light, the reflected light being the light irradiated from the first light emitting element 611 to the spun yarn 20 and reflected by the spun yarn 20. The second light receiving element 622 is arranged so as not to directly receive light from the second light emitting element 612 and arranged capable of receiving reflected light, the reflected light being the light irradiated from the second light emitting element 612 to the spun yarn 20 and reflected by the spun yarn 20.

In the above-described configuration, as illustrated in FIG. 3, when the first light emitting element 611 emits light, the first light receiving element 621 receives reflected light and the second light receiving element 622 receives transmitted light. A state of when the second light emitting element 612 emits light is not illustrated in the drawings; however, in this case, contrary to the above-described case, the first light receiving element 621 receives the transmitted light and the second light receiving element 622 receives the reflected light.

Since the above-described transmitted light is the light received by the light receiving elements 621 and 622 while being cut off by the spun yarn 20, intensity of the transmitted light changes accompanying a change in thickness of the spun yarn 20. Accordingly, by monitoring a change in the transmitted light received by the light emitting elements 621 and 622 in the analyzer 55, thickness unevenness of the spun yarn 20 can be detected.

Since the above-described reflected light is the light reflected on a surface of the spun yarn 20 and received by the light receiving elements 621 and 622, intensity of the above-described reflected light also changes according to the color of the surface of the spun yarn 20. Accordingly, by detecting a change in the reflected light received by such light receiving elements in the analyzer 55, a foreign particle included in the spun yarn 20 can be detected.

Particularly, since a yellow LED is selected as a light source in the first measuring section 51 according to the present embodiment, a blue foreign particle being complementary color of yellow can be detected at high sensitivity. Meanwhile, a yellow foreign particle is difficult to be detected by a yellow light source; however, such difficulty can be complemented by the use of the second measuring section 52.

Further, as the spun yarn 20 becomes thicker, a surface area of the spun yarn 20 by which light is reflected becomes wider. Intensity of the reflected light is thus increased. That is, the intensity of the reflected light from the spun yarn 20 is also influenced by the thickness of the spun yarn 20. However, the light receiving elements 621 and 622 also receive reflected light from portions other than the spun yarn 20 (e.g., reflected light from a wall surface of the yarn travelling pathway 49a) . Since intensity of the reflected light from the portion other than the spun yarn 20 becomes weaker as the spun yarn 20 becomes thicker, a change in the reflected light from the spun yarn 20 can be neutralized. Accordingly, since the intensity of the reflected light is not influenced by a change in yarn thickness, a foreign particle can be detected with high accuracy. Further, a reflective plate (not illustrated in the drawings) having appropriate reflectivity is fastened to the wall surface of the yarn travelling pathway 49a such that the change in the reflected light from the spun yarn 20 can be appropriately neutralized.

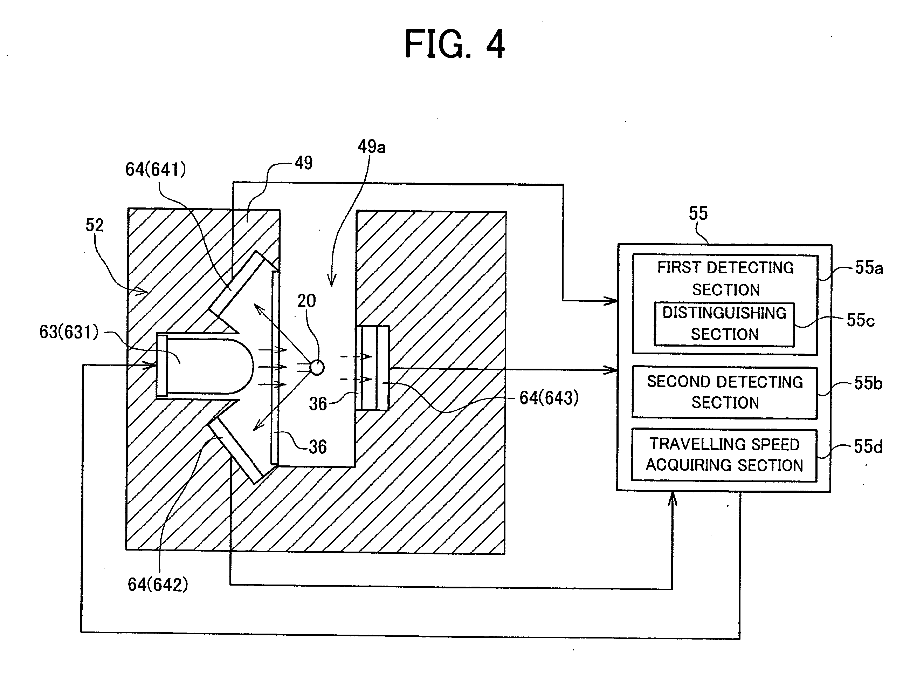

Next, the second measuring section 52 will be described. FIG. 4 is a schematic cross-sectional view illustrating a configuration of the second measuring section 52 inside the clearer head 49 of when the clearer head 49 is cut in a plane perpendicular to the yarn travelling direction.

The second measuring section 52 includes the second light source section 63 and the second light receiving section 64. As illustrated in FIG. 4, the second light source section 63 includes a third light emitting element 631 and the second light receiving section 64 includes three light receiving elements (a third light receiving element 641, a fourth light receiving element 642, and a fifth light receiving element 643). The third light emitting element 631, which is structured as a blue LED, irradiates blue light to the spun yarn 20 travelling along the yarn travelling pathway 49a. The light receiving elements 641, 642, and 643, which are structured as photodiodes, convert intensity of received light into an electric signal and transmit the electric signal to the analyzer 55. This type of measuring section is referred to as a single-axis measuring section since the measuring section is provided with a single light emitting element.

The yarn travelling pathway 49a is separated from the second light source section 63 and the second light receiving section 64 by the resinous transparent plate 36. The second light source section 63 irradiates light to the spun yarn 20 through the transparent plate 36, and the second light receiving section 64 receives light through the transparent plate 36.

The fifth light receiving element 643 is arranged opposite the third light emitting element 631 across a travelling path of the spun yarn 20. Accordingly, the fifth light receiving element 643 receives transmitted light, the transmitted light being the light irradiated from the third light emitting element 631 to the spun yarn 20 and transmitted through the spun yarn 20. Meanwhile, the third light receiving element 641 and the fourth light receiving element 642 are arranged so as not to receive the light directly from the third light emitting element 631, and arranged capable of receive reflected light, the reflected light being the light irradiated from the third light emitting element 631 to the spun yarn 20 and reflected by the spun yarn 20 . The third light receiving element 641 and the fourth light receiving element 642 are respectively arranged on each side of the third light emitting element 631 in a depth direction of the clearer head 49 (in a vertical direction of FIG. 4).

The fifth light receiving element 643 is arranged so as to be orthogonal to an optical axis. Accordingly, in the depth direction of the clearer head 49 (in the vertical direction of FIG. 4) , transmitted light can be more widely received by the single-axis measuring section than by the double-axis measuring section such as the first measuring section 51. As described above, since the single-axis measuring section can receive transmitted light widely, a detection range can be increased and different types of textile materials ranging up to an extra-thick material can be measured.

By monitoring a change in transmitted light received by the fifth light receiving element 643, the analyzer 55 can detect thickness unevenness of the spun yarn 20. By detecting a decrease in the amount of reflected light received by the third light receiving element 641 and the fourth light receiving element 642, the analyzer 55 can detect a foreign particle included in the spun yarn 20.

Particularly, since a blue LED is selected as a light source in the second measuring section 52 according to the present embodiment, a yellow foreign particle being complementary color of blue can be detected at high sensitivity. Meanwhile, a blue foreign particle is difficult to be detected by a blue light source; however, such difficulty can be complemented by the use of the first measuring section 51. Further, also in the second measuring section 52, in the same manner as the first measuring section 51, a reflective plate (not illustrated in the drawings) is arranged appropriately such that intensity of the reflected light received by the light receiving elements 641 and 642 is not influenced by yarn thickness.

Next, detection of a foreign particle by the analyzer 55 will be described. The analyzer 55 has a function serving as a first detecting section 55a adapted to detect a foreign particle in the spun yarn 20 in accordance with reflected light received by the first light receiving section 62 of the first measuring section 51 and reflected light received by the second light receiving section 64 of the second measuring section 52.

The first detecting section 55a compares each of measured values of the reflected light received by the first light receiving section 62 and the second light receiving section 64 with a threshold value to determine whether or not a foreign particle is included in the spun yarn 20. For example, when the spun yarn 20 is made of cotton fibers, the spun yarn 20 is white; therefore, the spun yarn 20 indicates high optical reflectivity. Meanwhile, a colored foreign particle indicates low optical reflectivity. Accordingly, when the colored foreign particle is included in the spun yarn 20, the amount of light reflected by the spun yarn 20 decreases. Therefore, in the first detecting section 55a, a foreign particle detecting threshold value is preliminary set to be smaller than a measured value of light to be reflected by the spun yarn 20 including no foreign particle. Then, when each of the measured values of the reflected light received by the first light receiving section 62 and the second light receiving section 64 becomes equal to or less than the foreign particle detecting threshold value, the first detecting section 55a determines that a foreign particle is included in the spun yarn 20. In the clearer 15 according to the present embodiment, since the first light receiving section 62 and the second light receiving section 64 receive reflected lights in complementary colors, foreign particles of all colors can be detected by taking into account the measured values obtained by both of the light receiving sections as described above. Further, optical reflectivity differs depending on the color of a light source; therefore, the foreign particle detecting threshold value to be compared with a measured result may be difference in the first light receiving section 62 and the second light receiving section 64.

Further, a foreign particle included in the spun yarn 20 dose not always cause a decrease in the amount of reflected light received by the first light receiving section 62 and the second light receiving section 64. For example, if a polypropylene fiber is mixed in the spun yarn 20 as a foreign particle, since polypropylene is white, there is little decrease in the amount of reflected light. Accordingly, a conventional clearer had difficulty in detecting polypropylene. Even if polypropylene is mixed in a white spun yarn 20, a portion in which polypropylene is mixed is unnoticeable. However, when the white spun yarn 20 is dyed in a later process, the color of the portion in which polypropylene is mixed remains white without being dyed, which causes a problem that polypropylene which is white is extremely noticeable.

In this regard, in the clearer 15 according to the present embodiment, since the second measuring section 52 uses blue light, polypropylene can be detected. That is, polypropylene has a property which indicates higher reflectivity with respect to the blue light than a property of a cotton fiber. Accordingly, when polypropylene is mixed in the spun yarn 20 made of cotton fibers, reflection of blue light is rather increased. Therefore, the first detecting section 55a preliminary sets a polypropylene detecting threshold value that is larger than a measurement value of blue light to be reflected by the spun yarn 20 including no foreign particle. When the measured value of the reflected light received by the second light receiving section 64 indicates at least the polypropylene detecting threshold value, the first detecting section 55a also determines that a foreign particle is included in the spun yarn 20. Consequently, the clearer 15 can remove polypropylene as a foreign particle.

Meanwhile, as disclosed in Japanese Unexamined Patent Application Publication No. 2007-212423, a trash such as a cotton stem or a leave becomes unnoticeable by being dyed along with cotton yarns in a later process. Therefore, such a trash is a foreign particle which is not required to be removed. If such a foreign particle which is not required to be removed is removed, efficiency of forming a package by the winder unit 10 is decreased. In order to prevent such a trash from being removed, the first detecting section 55a is provided with a distinguishing section 55c adapted to determine whether or not the foreign particle is a trash.

When a foreign particle is detected, the distinguishing section 55c determines whether or not such a foreign particle is yellow. That is, as disclosed in Japanese Unexamined Patent Application Publication No. 2007-212423, since the color of a trash is yellow or yellowish orange, a foreign particle determined to be yellow is likely a trash. Specifically, if a foreign particle is not detected from a measurement result of the first light receiving section 62 receiving reflection of yellow light and a foreign particle is detected from a measurement result of the second light receiving section 64 receiving reflection of blue light, the distinguishing section 55c determines that the color of such a foreign particle is yellow.

Further, there is also a possibility that a yellow foreign particle other than a trash is included in the spun yarn 20. Although the clearer 15 is required to remove the trash, the clearer 15 is required to remove the yellow foreign particle other than the trash. In many cases, the yellow foreign particle other than the trash is a yellow fiber fragment (referred to as a colored yarn) which was mixed during a different process.

Therefore, the distinguishing section 55c determines whether the yellow foreign particle is a trash or a colored yarn. Further, the trash is characterized by "being dark in color and being short in length", and the colored yarn is characterized by "being light in color and being long in length". Accordingly, by irradiating blue light to spun yarn 20, the trash can be detected as a foreign particle characterized by "being dark in color and being short in length", and the colored yarn can be detected as a foreign particle characterized by "being light in color and being long in length". In view of the above-described characteristic features, the distinguishing section 55c sets a threshold value for the length of a yellow foreign particle, and determines that the yellow foreign particle is the colored yarn only when detecting the yellow foreign particle that is longer than the threshold value. In place of such a configuration or in addition to such a configuration, the distinguishing section 55c may set a threshold value for color density of the yellow foreign particle, and may determine that the foreign particle is the colored yarn only when detecting the yellow foreign particle that is lighter than the threshold value.

Further, when yellow light is irradiated to the spun yarn 20, the yellow foreign particle cannot be detected; therefore, the color density or the length of the yellow foreign particle cannot be also detected. Only by irradiating colored light other than yellow light to the spun yarn 20 in the same manner as the clearer 15 according to the present embodiment, the color density or the length of a yellow foreign particle can be detected. Accordingly, by a configuration provided only with yellow light as described in Japanese Unexamined Patent Application Publication No. 2007-212423, a determination could not be made whether the yellow foreign particle is a trash or a yellow yarn.

As described above, by detecting a foreign particle by a combination of yellow light and blue light, the clearer 15 according to the present embodiment can remove a yellow foreign particle other than a trash while not leaving the trash. Further, since the clearer 15 according to the present embodiment detects a foreign particle by the use of blue light, polypropylene can be detected as described above.

Next, detection of thickness unevenness of a yarn by the analyzer 55 will be described. The analyzer 55 has a function serving as a second detecting section 55b adapted to detect thickness unevenness of the spun yarn 20 in accordance with transmitted light received by the second light receiving section 64 of the second measuring section 52.

The second detecting section 55b monitors a measured value of the transmitted light received by the second light receiving section 64, and when the change in the measured value exceeds an acceptable range, determines that the thickness of the spun yarn 20 is abnormal.

The analyzer 55 also functions as a speed acquiring section (travelling speed acquiring section) 55d which acquires travelling speed of the spun yarn 20 in accordance with the transmitted light received by the first light receiving section 62 and the second light receiving section 64. That is, in the clearer 15 according to the present embodiment, the first measuring section 51 is arranged at prescribed interval at upstream of the second measuring section 52 in the yarn travelling direction. Accordingly, a measured result of transmitted light received by the second light receiving section 64 is output later than a measured result of transmitted light received by the first light receiving section 62. Such a time delay is proportional to the travelling speed of the spun yarn 20.

The speed acquiring section 55d detects how long output of the measured result of the transmitted light received by the second light receiving section 64 is delayed compared with output of the measured result of the transmitted light received by the first light receiving section 62, and then acquires the travelling speed of the spun yarn 20 in accordance with such a detection result and the above-described prescribed interval. In the clearer 15 according to the present embodiment, the measuring sections 51 and 52 for performing measurement by the use of light sources of two different colors are arranged one above the other. Such a configuration in which the measuring sections 51 and 52 are arranged one above the other is also used to detect yarn travelling speed. The yarn travelling speed acquired as described above is transmitted to the unit control section 50 and the like, and used to control winding of the spun yarn 20.

As described above, the clearer 15 according to the present embodiment includes the first light source section 61, the second light source section 63, the first light receiving section 62, the second light receiving section 64, the first detecting section 55a, and the second detecting section 55b. The first light source section 61 irradiates yellow light to the spun yarn 20. The second light source section 63 irradiates blue light to the spun yarn 20, the blue light being complementary color of yellow. The first light receiving section 62 receives reflected light, the reflected light being the light from the first light source section 61 reflected by the spun yarn 20. The second light receiving section 64 receives reflected light and transmitted light, the reflected light being the light from the second light source section 63 reflected by the spun yarn 20, and the transmitted light being the light from the second light source section 63 that has been transmitted through the spun yarn 20. The first detecting section 55a detects a foreign particle in the spun yarn 20 in accordance with the reflected light received by the first light receiving section 62 and the second light receiving section 64. The second detecting section 55b detects thickness of the spun yarn 20 in accordance with the transmitted light received by the second light receiving section 64.

As described above, by the use of light of two different colors of which one is complementary color with respect to the other, even if sensitivity for detecting a foreign particle of a particular color is reduced by light from one of the two light sources, such a foreign particle can be detected by light from the other light source. Since weakness with respect to particular colors can be complemented by the two light sources as described above, the clearer 15 according to the present embodiment can detect foreign particles of all colors at excellent sensitivity. Further, by using one of the two light sources as a light source for detecting yarn thickness, the clearer 15 can be structured to be simple so that costs can be reduced. Since polypropylene has a property indicating high optical reflectivity with respect to blue light, the clearer 15 can detect polypropylene by detection of a foreign particle by the use of a blue light source as described above. By detecting a foreign particle by the use of a yellow light source being complementary color of blue, the clearer 15 can successfully detect a blue foreign particle which is difficult to be detected by the blue light source.

In the clearer 15 according to the present embodiment, the first detecting section 55a includes the distinguishing section 55c adapted to compare a measured value of reflected light with a threshold value to distinguish a trash and a foreign particle, other than the trash, mixed in the spun yarn 20. Since the trash has a color which is difficult to be detected by a yellow light source, by comparing reflected light from the yellow light source with reflected light from a light source other than the yellow light source, the clearer 15 can distinguish whether or not the detected foreign particle is a trash.

The clearer 15 according to the present embodiment includes the clearer head 49 which is a resinous housing adapted to accommodate the first light source section 61, the second light source section 63, the first light receiving section 62, and the second light receiving section 64. Since visible light selected in the above-described embodiment does not deteriorate resin, the first light source section 61 and the second light source section 63 can be accommodated in the resinous housing. By accommodating the two light source sections and the two light receiving sections in a single housing, the clearer 15 can be formed compact.

In the clearer 15 according to the present embodiment, the first light receiving section 62 receives transmitted light in addition to the reflected light, the transmitted light being the light from the first light source section 61 that has been transmitted through the spun yarn 20. In the travelling direction of the spun yarn 20, the first light source section 61 and the first light receiving section 62 are arranged at prescribed interval at upstream of the second light source section 63 and the second light receiving section 64. The clearer 15 includes the speed acquiring section 55d adapted to acquire travelling speed of the spun yarn 20 in accordance with transmitted light received by the first light receiving section 62 and the second light receiving section 64 and the above-described prescribed interval. When measuring light transmitted through the travelling spun yarn 20 at upstream of the travelling direction of the spun yarn 20 and at downstream thereof, there is a time delay in a measured value at downstream thereof when comparing with a measured value at upstream thereof. Therefore, the clearer 15 can calculate the travelling speed of the spun yarn 20 by detecting such a time delay.

In the clearer 15 according to the present embodiment, the first light source section 61 includes the two light emitting elements 611 and 612 that alternately emit light. The first light receiving section 62 includes two light receiving elements 621 and 622 that receive reflected light and transmitted light. The second light source section 63 includes a single light emitting element 631. The second light receiving section 64 includes a single light receiving element 643 that receives transmitted light.

The first light source section 61 and the first light receiving section 62 are structured as a double-axis measuring section, and the second light source section 63 and the second light receiving section 64 are structured as a single-axis measuring section. The double-axis measuring section can irradiate light to the spun yarn 20 from a plurality of directions; therefore, excellent detection sensitivity can be maintained even if a cross-sectional shape of the spun yarn 20 is flat. Meanwhile, since the light receiving element 643 perpendicular to a light axis is arranged in the single-axis measuring section, detection range of transmitted light is wide. Each of the single-axis measuring section and the double-axis measuring section has a feature described above; therefore, by combining such two types of measuring sections, detection performance of the clearer 15 can be improved.

The automatic winder according to the present embodiment includes the winding section 31 adapted to wind a spun yarn into a package and the clearer 15 adapted to measure the spun yarn that is travelling. Accordingly, the automatic winder can form a high-quality package in which a foreign particle is removed and an abnormality in yarn thickness is solved.

Next, a modification according to the above-described embodiment will be described. Further, in the following description of the modification, the same reference numerals are denoted for the configurations identical to or similar to those of the above-described embodiment, and the description thereof will be omitted.

In the modification, in the second measuring section 52, an ultraviolet LED is used as a second light source section 63 in place of the blue LED. That is, since polypropylene has a property indicating higher reflectivity with respect to ultraviolet light than blue visible light, sensitivity for detecting polypropylene can be further improved by adapting the ultraviolet LED.

Since the spectrum of ultraviolet light is similar to the spectrum of blue, the ultraviolet light indicates high sensitivity for detecting a yellow foreign particle which is complementary color of blue, but indicates low sensitivity for detecting a blue foreign particle. Therefore, also in the modification, a yellow LED is used as the first light source section 61 as in the above-described embodiment. Accordingly, a clearer according to the modification also can detect foreign particles of various colors.

Further, since ultraviolet light deteriorates resin, a resinous transparent plate 36 cannot be used in the clearer head 49. Therefore, in the present embodiment, a transparent plate 36 made of quartz is used in the clearer head 49. Since the above-described embodiment can use a cost-efficient resinous transparent plate 36, the above-described embodiment using only visible light is preferable.

As described above, the clearer 15 according to the present modification includes the first light source section 61, the second light source section 63, the first light receiving section 62, the second light receiving section 64, the first detecting section 55a, and the second detecting section 55b. The first light source section 61 irradiates yellow light to the spun yarn 20. The second light source section 63 irradiates ultraviolet light to the spun yarn 20. The first light receiving section 62 receives reflected light, the reflected light being the light from the first light source section 61 reflected by the spun yarn 20. The second light receiving section 64 receives reflected light and transmitted light, the reflected light being the light from the second light source section 63 reflected by the spun yarn 20, and the transmitted light being the light from the second light source section 63 that has been transmitted through the spun yarn 20. The first detecting section 55a detects a foreign particle in the spun yarn 20 in accordance with the reflected light received by the first light receiving section 62 and the second light receiving section 64. The second detecting section 55b detects thickness of the spun yarn 20 in accordance with the transmitted light received by the second light receiving section 64.

That is, since polypropylene has a property indicating high reflectivity with respect to ultraviolet light, polypropylene can be detected using a light source of ultraviolet light as described above. Meanwhile, it is difficult to detect a blue foreign particle by the above-described light source of ultraviolet light which is similar to blue light in wavelength. Accordingly, by detecting a foreign particle by the use of a yellow light source being complementary color of blue, a blue foreign particle which is difficult to be detected by the ultraviolet light can be detected.

The preferred embodiment and the modification thereof have been described above; however, configurations of the embodiment and the modification can be, for example, modified as follows.

Although a description has been made by specifying the color by the name of light such as "yellow" or "blue", the description is not intended to be limited to such colors, and the specified colors also include similar colors. For example, when a yellow LED is referred in general, the yellow LED also includes an LED which emits color similar to orange (e.g., yellowish orange). Such a yellowish orange light is also regarded as yellow light in the present description.

Since any color combination can be adapted as two colors in which one is complementary color with respect to the other, for example, a color combination of red and green can be selected in place of a color combination of yellow and blue as in the above-described embodiment. Since a red LED and a green LED can easily be obtained, the clearer can be manufactured economically.

Although in the above-described embodiment, the first measuring section 51 is structured as a double-axis measuring section, and the second measuring section 52 is structured as a single-axis measuring section, , the first measuring section 51 may be structured as the single-axis measuring section, and the second measuring section 52 may be structured as the double-axis measuring section. Further, instead of a combination of the single-axis measuring section and the double-axis measuring section, both of the measuring sections 51 and 52 may be structured as the single-axis measuring section or the double-axis measuring section.

A light source is not limited to a LED, and may be replaced with an appropriate white light source provided with a colored filter, for example.

Although the transmitted light is measured by both the first measuring section 51 and the second measuring section 52 in the above-described embodiment, the transmitted light may be measured only by one of the measuring sections for detecting yarn thickness. Accordingly, when yarn travelling speed is not required to be obtained, the yarn thickness is not required to be detected by both of the measuring sections.

A textile material measured by the textile material measuring device is not limited to a spun yarn. For example, a sliver may be used. Even in this case, foreign particles of various colors included in the sliver can be detected at high sensitivity.

In the above-described embodiment, while a package is being rotated by the rotating winding drum 24, a yarn is traversed on a surface of the package. However, the present invention is not limited to the above-described embodiment. A configuration of the present invention can be applied even to a yarn winding machine in which driving of a package and traversing is separately carried out. For example, as such a yarn winding machine, the following example is considered: an automatic winder including an arm-typed traverse device adapted to traverse a yarn by a swinging arm or a belt-typed traverse device adapted to traverse a yarn by a yarn hooking member being reciprocated by a belt, or the like. In this case, the package may be driven and rotated directly by a motor in order to drive and rotate a contact roller which is not provided with a traversing mechanism.

The configuration of the present invention can be applied not only to the automatic winder but to a different type of yarn winding machine such as a spinning machine.

## Claims

1. A textile material measuring device comprising:
a first light source section (61) adapted to irradiate first light to a textile material;
a second light source section (63) adapted to irradiate second light to the textile material;
a first light receiving section (62) adapted to receive reflected light, the reflected light being the light from the first light source section (61) reflected by the textile material (20);
a second light receiving section (64) adapted to receive reflected light and transmitted light, the reflected light being the light from the second light source section (63) reflected by the textile material (20), and the transmitted light being the light from the second light source section (63) that has been transmitted through the textile material;
a first detecting section (55a) adapted to detect a foreign particle in the textile material in accordance with the reflected light received by the first light receiving section (62) and the second light receiving section (64); and
a second detecting section (55b) is adapted to detect thickness of the textile material in accordance with the transmitted light received by the second light receiving section (64),
**characterized in that**
the first light is first-color light and the second light is second-color light being complementary color with respect to the first-color light or wherein the first light is yellow light and the second light is ultraviolet light.

2. The textile material measuring device according to claim 1, wherein the first light is first-color light and the second light is second-color light being complementary color with respect to the first-color light, wherein the first-color of the light irradiated by the first light source section (61) is yellow, and the second-color of the light irradiated by the second light source is blue.

3. The textile material measuring device according to claim 1 or claim 2, wherein the first detecting section (55a) includes a distinguishing section (55c) adapted to compare a measured value of the reflected light with a threshold value to distinguish a trash and a foreign particle, other than the trash, mixed in the textile material.

4. The textile material measuring device according to one of claims 1 to 3, wherein the first light is first visible-color light and the second light is second visible-color light, the textile material measuring device further comprising a resinous housing (49) adapted to accommodate the first light source section (61), the second light source section (63), the first light receiving section (62), and the second light receiving section (64) .

5. The textile material measuring device according to any one of claim 1 through claim 4, wherein
the first light receiving section (62) is adapted to receive transmitted light in addition to the reflected light, the transmitted light being the light from the first light source section (61) transmitted through the textile material,
in a travelling direction of the textile material, the first light source section (61) and the first light receiving section (62) are arranged at a prescribed interval at upstream of the second light source section (63) and the second light receiving section (64), and
a travelling speed acquiring section (55d) is further provided to acquire travelling speed of the textile material in accordance with the transmitted light received by the first light receiving section (62) and the second light receiving section (64) and the prescribed interval.

6. The textile material measuring device according to any one of claim 1 through claim 5, wherein the first light source section (61) includes two light emitting elements (611, 612) that alternately emit light, the first light receiving section (62) includes two light receiving elements (621, 622) that receive reflected light and transmitted light, the second light source section (63) includes one light emitting element (631), and the second light receiving section (64) includes one light receiving element (643) that receives transmitted light.

7. A yarn winding machine comprising:
a winding device (31) adapted to wind yarn into a package; and
the textile material measuring device (15) according to any one of claim 1 through claim 6 adapted to measure the yarn that is travelling.

## Patentansprüche

1. Ein Textilmaterialmessvorrichtung, die folgende Merkmale aufweist:
einen ersten Lichtquellenabschnitt (61), der dazu angepasst ist, ein erstes Licht auf ein Textilmaterial auszustrahlen;
einen zweiten Lichtquellenabschnitt (63), der dazu angepasst ist, ein zweites Licht auf das Textilmaterial auszustrahlen;
einen ersten Lichtempfangsabschnitt (62), der dazu angepasst ist, reflektiertes Licht zu empfangen, wobei das reflektierte Licht das Licht von dem ersten Lichtquellenabschnitt (61) ist, das durch das Textilmaterial (20) reflektiert wird;
einen zweiten Lichtempfangsabschnitt (64), der dazu angepasst ist, reflektiertes Licht und durchgelassenes Licht zu empfangen, wobei das reflektierte Licht das Licht von dem zweiten Lichtquellenabschnitt (63) ist, das durch das Textilmaterial (20) reflektiert wird, ist, und das durchgelassene Licht das Licht von dem zweiten Lichtquellenabschnitt (63) ist, das durch das Textilmaterial durchgelassen wird;
einen ersten Erfassungsabschnitt (55a), der dazu angepasst ist, gemäß dem reflektierten Licht, das durch den ersten Lichtempfangsabschnitt (62) und den zweiten Lichtempfangsabschnitt (64) empfangen wird, ein Fremdpartikel in dem Textilmaterial zu erfassen; und
einen zweiten Erfassungsabschnitt (55b), der dazu angepasst ist, gemäß dem durchgelassenen Licht, das durch den zweiten Lichtempfangsabschnitt (64) empfangen wird, die Dicke des Textilmaterials zu erfassen,
**dadurch gekennzeichnet, dass**
das erste Licht ein Licht erster Farbe ist und das zweite Licht ein Licht zweiter Farbe ist, das bezüglich des Lichts erster Farbe eine komplementäre Farbe aufweist, oder wobei das erste Licht gelbes Licht ist und das zweite Licht ultraviolettes Licht ist.

2. Die Textilmaterialmessvorrichtung gemäß Anspruch 1, bei der das erste Licht ein Licht erster Farbe ist und das zweite Licht ein Licht zweiter Farbe ist, das bezüglich des Lichts erster Farbe eine komplementäre Farbe aufweist, wobei die erste Farbe des durch den ersten Lichtquellenabschnitt (61) ausgestrahlten Lichts gelb ist und die zweite Farbe des durch die zweite Lichtquelle ausgestrahlten Lichts blau ist.

3. Die Textilmaterialmessvorrichtung gemäß Anspruch 1 oder 2, bei der der erste Erfassungsabschnitt (55a) einen Unterscheidungsabschnitt (55c) aufweist, der dazu angepasst ist, einen gemessenen Wert des reflektierten Lichts mit einem Schwellwert zu vergleichen, um einen Spinnereiabfall und ein Fremdpartikel, das kein Spinnereiabfall ist und das in das Textilmaterial gemischt ist, zu unterscheiden.

4. Die Textilmaterialmessvorrichtung gemäß einem der Ansprüche 1 bis 3, bei der das erste Licht ein Licht einer ersten sichtbaren Farbe ist und das zweite Licht ein Licht einer zweiten sichtbaren Farbe ist, wobei die Textilmaterialmessvorrichtung ferner ein harzhaltiges Gehäuse (49) aufweist, das dazu angepasst ist, den ersten Lichtquellenabschnitt (61), den zweiten Lichtquellenabschnitt (63), den ersten Lichtempfangsabschnitt (62) und den zweiten Lichtempfangsabschnitt (64) zu beherbergen.

5. Die Textilmaterialmessvorrichtung gemäß einem der Ansprüche 1 bis 4, bei der
der erste Lichtempfangsabschnitt (62) dazu angepasst ist, zusätzlich zu dem reflektierten Licht durchgelassenes Licht zu empfangen, wobei das durchgelassene Licht das Licht von dem ersten Lichtquellenabschnitt (61) ist, das durch das Textilmaterial durchgelassen wird,
in einer Laufrichtung des Textilmaterials der erste Lichtquellenabschnitt (61) und der erste Lichtempfangsabschnitt (62) in einem vorgeschriebenen Abstand in Verarbeitungsrichtung vor dem zweiten Lichtquellenabschnitt (63) und dem zweiten Lichtempfangsabschnitt (64) angeordnet sind, und
ein Laufgeschwindigkeitserfassungsabschnitt (55d) ferner dazu vorgesehen ist, die Laufgeschwindigkeit des Textilmaterials gemäß dem durchgelassenen Licht, das seitens des ersten Lichtempfangsabschnitts (62) und des zweiten Lichtempfangsabschnitts (64) empfangen wird, und dem vorgeschriebenen Abstand zu erfassen.

6. Die Textilmaterialmessvorrichtung gemäß einem der Ansprüche 1 bis 5, bei der der erste Lichtquellenabschnitt (61) zwei Lichtemissionselemente (611, 612) umfasst, die abwechselnd Licht emittieren, der erste Lichtempfangsabschnitt (62) zwei Lichtempfangselemente (621, 622) umfasst, die reflektiertes Licht und durchgelassenes Licht empfangen, der zweite Lichtquellenabschnitt (63) ein Lichtemissionselement (631) umfasst und der zweite Lichtempfangsabschnitt (64) ein Lichtempfangselement (643) umfasst, das durchgelassenes Licht empfängt.

7. Eine Garnaufwickelmaschine, die folgende Merkmale aufweist:
eine Aufwickelvorrichtung (31), die dazu angepasst ist, Garn zu einem Garnkörper aufzuwickeln; und
die Textilmaterialmessvorrichtung (15) gemäß einem der Ansprüche 1 bis 6, die dazu angepasst ist, das Garn, das gerade läuft, zu messen.

## Revendications

1. Dispositif de mesure de matériau textile, comprenant:
un premier segment de source de lumière (61) adapté pour irradier une première lumière sur un matériau textile;
un deuxième segment de source de lumière (63) adapté pour irradier une deuxième lumière sur le matériau textile;
un premier segment de réception de lumière (62) adapté pour recevoir la lumière réfléchie, la lumière réfléchie étant la lumière du premier segment de source de lumière (61) réfléchie par le matériau textile (20);
un deuxième segment de réception de lumière (64) adapté pour recevoir la lumière réfléchie et la lumière transmise, la lumière réfléchie étant la lumière du deuxième segment de source de lumière (63) réfléchie par le matériau textile (20), et la lumière transmise étant la lumière du deuxième segment de source de lumière (63) qui a été transmise à travers le matériau textile;
un premier segment de détection (55a) adapté pour détecter une particule étrangère dans le matériau textile en fonction de la lumière réfléchie reçue par le premier segment de réception de lumière (62) et le deuxième segment de réception de lumière (64); et
un deuxième segment de détection (55b) est adapté pour détecter l'épaisseur du matériau textile en fonction de la lumière transmise reçue par le deuxième segment de réception de lumière (64),
**caractérisé par le fait que**
la première lumière est une lumière d'une première couleur et la deuxième lumière est une lumière d'une deuxième couleur qui est une couleur complémentaire par rapport à la lumière d'une première couleur ou dans lequel la première lumière est une lumière jaune et la deuxième lumière est une lumière ultraviolette.

2. Dispositif de mesure de matériau textile selon la revendication 1, dans lequel la première lumière est une lumière d'une première couleur et la deuxième lumière est une lumière d'une deuxième couleur qui est une couleur complémentaire par rapport à la lumière d'une première couleur, dans lequel la première couleur de la lumière irradiée par le premier segment de source de lumière (61) est jaune, et la deuxième couleur de la lumière irradiée par la deuxième source de lumière est bleue.

3. Dispositif de mesure de matériau textile selon la revendication 1 ou la revendication 2, dans lequel le premier segment de détection (55a) comporte un segment de distinction (55c) adapté pour comparer une valeur mesurée de la lumière réfléchie avec une valeur de seuil pour distinguer les déchets de filature et une particule étrangère autre que les déchets de filature qui est mélangée dans le matériau textile.

4. Dispositif de mesure de matériau textile selon l'une des revendications 1 à 3, dans lequel la première lumière est une lumière d'une première couleur visible et la deuxième lumière est une lumière d'une deuxième couleur visible, le dispositif de mesure de matériau textile comprenant par ailleurs un boîtier en résine (49) adapté pour abriter le premier segment de source de lumière (61), le deuxième segment de source de lumière (63), le premier segment de réception de lumière (62) et le deuxième segment de réception de lumière (64).

5. Dispositif de mesure de matériau textile selon l'une quelconque de la revendication 1 à la revendication 4, dans lequel
le premier segment de réception de lumière (62) est adapté pour recevoir la lumière transmise en plus de la lumière réfléchie, la lumière transmise étant la lumière du premier segment de source de lumière (61) transmise à travers le matériau textile,
dans une direction de déplacement du matériau textile, le premier segment de source de lumière (61) et le premier segment de réception de lumière (62) sont disposés à un intervalle prescrit à l'amont du deuxième segment de source de lumière (63) et du deuxième segment de réception de lumière (64), et
un segment d'acquisition de vitesse de déplacement (55d) est par ailleurs prévu pour acquérir la vitesse de déplacement du matériau textile en fonction de la lumière transmise reçue par le premier segment de réception de lumière (62) et le deuxième segment de réception de lumière (64) et de l'intervalle prescrit.

6. Dispositif de mesure de matériau textile selon l'une quelconque de la revendication 1 à la revendication 5, dans lequel le premier segment de source de lumière (61) comporte deux éléments émetteurs de lumière (611, 612) qui émettent en alternance de la lumière, le premier segment de réception de lumière (62) comporte deux éléments de réception de lumière (621, 622) qui reçoivent la lumière réfléchie et lumière transmise, le deuxième segment de source de lumière (63) comporte un élément émetteur de lumière (631), et le deuxième segment de réception de lumière (64) comporte un élément de réception de lumière (643) qui reçoit la lumière transmise.

7. Machine de bobinage de fil, comprenant:
un dispositif de bobinage (31) adapté pour bobiner le fil pour obtenir une bobine; et
le dispositif de mesure de matériau textile (15) selon l'une quelconque de la revendication 1 à la revendication 6 adapté pour mesurer le fil qui se déplace.
